# EUROPEAN PATENT SPECIFICATION

(11) **EP 3 369 437 B1**
(45) Date of publication and mention of the grant of the patent: **17.02.2021**
(21) Application number: 17159043.3
(22) Date of filing: 03.03.2017
(51) Int. Cl.: A61L 2/22, A61L 2/20

(54) **APPARATUS AND METHOD FOR SANITIZING OF AN ELECTROMEDICAL DEVICE USING NEBULISED HYDROGEN PEROXIDE**
VORRICHTUNG UND VERFAHREN ZUR ENTKEIMUNG EINER ELEKTROMEDIZINISCHEN VORRICHTUNG MITTELS VERNEBELTEN WASSERSTOFFPEROXID
APPAREIL ET PROCÉDÉ DE DÉSINFECTION D'UN DISPOSITIF ÉLECTROMÉDICAL EN UTILISANT PÉROXYDE D'HYDROGÈNE NÉBULISÉ

(43) Date of publication of application: 05.09.2018
(73) Proprietor: SOL S.p.A., 20900 Monza (MB) (IT)
(72) Inventor: Valtolina, Daniele, 23896 Sirtori (IT)
(74) Representative: Ter Meer Steinmeister & Partner

(56) References cited:
- EP-B1- 2 323 701
- WO-A1-2011/104508
- WO-A1-2016/199733
- US-A- 5 645 796
- US-A1- 2013 216 438
- ANDERSEN ET AL: "Decontamination of rooms, medical equipment and ambulances using an aerosol of hydrogen peroxide disinfectant", JOURNAL OF HOSPITAL INFECTION, ELSEVIER, AMSTERDAM, NL, vol. 62, no. 2, 1 February 2006 (2006-02-01), pages 149-155, XP005235626, ISSN: 0195-6701, DOI: 10.1016/J.JHIN.2005.07.020

## Description

### FIELD

Embodiments of the present disclosure relate to an apparatus and a method for sanitizing of an electromedical device, and the use of a sanitizing product having hydrogen peroxide for sanitizing of an electromedical device. The present disclosure particularly relates to a sanitizing apparatus and a sanitization method using hydrogen peroxide to sanitize contaminated electromedical devices, such as medical ventilators.

### BACKGROUND

Electromedical devices, such as medical ventilators, can be complex and expensive devices and it is thus beneficial to reuse such electromedical devices. In order to avoid microbial cross-contamination, the electromedical devices can be disinfected using, for example, a sanitization method. Some known sanitization methods do only clean surfaces, e.g., outer surfaces, of the electromedical device, and the disinfection of the electromedical device is incomplete. Other sanitization methods use ozone in a sanitization process. Ozone is dangerous to health and is a strong oxidant that can damage the electromedical device, and particularly electronics thereof.

In view of the above, an apparatus, a method and a sanitizing product for sanitizing of an electromedical device, that overcome at least some of the problems in the art are beneficial. The present disclosure particularly aims at providing an apparatus, a method, and a sanitizing product that can improve a sanitizing efficiency of a sanitization process. The present disclosure further aims at providing an apparatus, a method and a sanitizing product that can sanitize the electromedical device without damaging the electromedical device, and particularly the electronics thereof.

US 2013/0216438 A1 describes an apparatus for decontaminating non-critical medical devices, such as wheelchairs, IV poles, blood pressure cuffs, case carts and other devices encountered in a hospital environment. A vaporized hydrogen peroxide generator is provided on the exterior of an enclosure. The VHP generator is an apparatus for generating vaporized hydrogen peroxide from liquid hydrogen peroxide.

EP 2323701 A4 describes a disinfection system for any objects. The aerosol flow into the chamber is typically provided by means of an outside ultrasonic nebuliser that provides a square wave dosage pattern.

US 5,645796 A describes a plasma sterilizing process using a magnetron that is energized to create a plasma, wherein the plasma flows into the sterilizing chamber.

WO2011/104508 A1 describes a method of rendering harmless chemical and/or biological warfare agents on a surface, comprising: exposing the agents to an atmosphere comprising peroxide vapour, and causing the peroxide vapour to condense on the surface having the chemical and/or biological agents thereon; after the first step exposing the agents to an atmosphere comprising further peroxide vapour, and causing the further peroxide vapour to condense on the surface having the chemical and/or biological agents thereon; and optionally repeating the second step.

WO 2016/199733 A1 describes a sterilization device with a sterilization chamber in which an object to be sterilized is placed; a door for opening and closing the sterilization chamber; and a sensor for detecting an obstacle present in the direction of movement of the door when the door is closing. The sterilization device is configured so that when the sensor detects the obstacle, the movement of the door is stopped or the door is moved in a direction opposite to the direction the door was moving.

An article by Andersen et al. ("Decontamination of rooms, medical equipment and ambulances using an aerosol of hydrogen peroxide disinfectant", Journal of Hospital Infection, 62, pp. 149-155, 2006) suggests a method for decontamination in which a H₂O₂ dry fumigation system is placed in various positions in rooms, ambulances and inside different types of medical equipment.

### SUMMARY

In light of the above, an apparatus, a method and a sanitizing product for sanitizing of an electromedical device are provided. Further aspects, benefits, and features of the present disclosure are apparent from the claims, the description, and the accompanying drawings.
The object is solved by the features of the independent claims. Preferred embodiments are given in the dependent claims.

According to an aspect of the present disclosure, an apparatus for sanitizing of an electromedical device is provided. The apparatus includes a housing having a reception space configured for receiving the electromedical device, wherein the reception space has a volume, at least one nebulizer device configured for nebulizing a sanitizing product, wherein the at least one nebulizer is located inside the reception space for introducing the nebulized sanitizing product into the reception space, and a controller configured for controlling the at least one nebulizer device for introducing a plurality of doses of the nebulized sanitizing product into the reception space during a sanitization process.

The controller is configured to introduce the plurality of doses at predetermined time intervals into the reception space.

Preferably, the predetermined time intervals are between 90 seconds and 210 seconds, and particularly between 120 seconds and 180 seconds.

Preferably, the controller is configured to perform the sanitization process for a duration between 30 minutes and 90 minutes, and particularly for a duration of about 60 minutes.

Preferably, the reception space has a volume of 5m³ or less or 3m³ or less, particularly wherein the volume is between 2 m ³ and 3 m³

Preferably, the housing has at least one opening in the reception space and at least one door configured for closing the opening.

Preferably, the housing further includes a first opening in the reception space and a second opening in the reception space; and a first door configured for closing the first opening and a second door configured for closing the second opening, wherein the first opening and the second opening are provided on opposite or adjacent sides of the reception space.

The apparatus, further includes an electrical connection port in the reception space, wherein the electrical connection port is configured for being connected with the electromedical device to operate the electromedical device during the sanitization process.

The sanitizing product is a hydrogen peroxide based sanitizing product, the hydrogen peroxide concentration is between 4% and 10%.

Preferably, each dose of the plurality of doses has between 0.5ml and 25ml of the sanitizing product per m³ of the volume of the reception space, and particularly between 15ml and 20ml of the sanitizing product per m³ of the volume of the reception space.

According to another aspect of the present disclosure, a method for sanitizing of an electromedical device is provided. The method includes a nebulizing of a sanitizing product, and an introducing of a plurality of doses of the nebulized sanitizing product into a reception space having the electromedical device therein.

Preferably, the hydrogen peroxide concentration is between 6% and 8%, and particularly the hydrogen peroxide concentration is 7.9%.

Introducing the plurality of doses into the reception space includes: introducing the plurality of doses in predetermined time intervals, wherein preferably the predetermined time intervals are between 90 seconds and 210 seconds, and particularly between 120 seconds and 180 seconds.

Preferably, the sanitizing of the electromedical device is performed for a duration between 30 minutes and 90 minutes, and particularly for a duration of about 60 minutes.

Preferably, the sanitizing of the electromedical device is performed at a temperature between 10°C and 40°C, and particularly at a temperature between 15°C and 30°C.

Preferably, the sanitizing of the electromedical device is performed at a relative humidity between 10% and 90%, and particularly at a relative humidity between 25% and 75%.

Preferably, the sanitizing of the electromedical device is performed at a pressure between 900mbar and 1100mbar, particularly at a pressure between 1005mbar and 1020mbar, and more particularly at atmospheric pressure.

The electromedical device 10) is operated during the sanitizing thereof.

Another aspect of the present disclosure relates to the use of a sanitizing product having a hydrogen peroxide concentration between 4% and 10%, and particularly between 6 and 8%, for sanitizing an electromedical device.

Embodiments are also directed at apparatuses for carrying out the disclosed methods and include apparatus parts for performing each described method aspect. These method aspects may be performed by way of hardware components, a computer programmed by appropriate software, by any combination of the two or in any other manner. Furthermore, embodiments according to the disclosure are also directed at methods for operating the described apparatus. The method includes aspects for carrying out every function of the apparatus.

### BRIEF DESCRIPTION OF THE DRAWINGS

So that the manner in which the above recited features of the present disclosure can be understood in detail, a more particular description of the disclosure, briefly summarized above, may be had by reference to embodiments. The accompanying drawings relate to embodiments of the disclosure and are described in the following:
- FIG. 1: shows a schematic perspective view of an apparatus (not falling under the scope of the claimed invention) for sanitizing of an electromedical device;
- FIG. 2: shows a schematic perspective view of an apparatus for sanitizing of an electromedical device according to the invention
- FIG. 3: shows a schematic top view of an apparatus for sanitizing of an electromedical device according to yet further embodiments described herein;
- FIGs. 4A, B: show schematic top views of the apparatus of FIG. 3;
- FIG. 5: shows a flow chart of a method for sanitizing of an electromedical device according to embodiments described herein; and
- FIG. 6: illustrates a sanitization process with multiple doses according to embodiments described herein.

### DETAILED DESCRIPTION OF EMBODIMENTS

Reference will now be made in detail to the various embodiments of the disclosure, one or more examples of which are illustrated in the figures. Within the following description of the drawings, the same reference numbers refer to same components. Generally, only the differences with respect to individual embodiments are described. Each example is provided by way of explanation of the disclosure and is not meant as a limitation of the disclosure. Further, features illustrated or described as part of one embodiment can be used on or in conjunction with other embodiments to yield yet a further embodiment. It is intended that the description includes such modifications and variations.

Electromedical devices should be sanitized or disinfected before they are reused e.g. for another patient in order to avoid microbial cross-contamination. The present disclosure uses a reception space in which the electromedical device to be cleaned is positioned, wherein a plurality of doses of a nebulized sanitizing product including, for example, hydrogen peroxide, are introduced into the reception space during a sanitization process. The reception space is closed during the sanitization process. The electromedical device, and specifically internal parts thereof, such as ventilators, internal pneumatic circuits, tubes, and outlet flow connectors of medical ventilators, can be disinfected using the nebulized sanitizing product, such as a dry-mist hydrogen peroxide based sanitizing product. The embodiments of the present disclosure can achieve a reduction of bacterial contamination of more than 3 Log in just one sanitization process or cycle.

In view of the above, a sanitizing efficiency of the sanitization process can be improved. Further the electromedical device can be disinfected without damaging the electromedical device, and particularly without damaging electronics thereof.

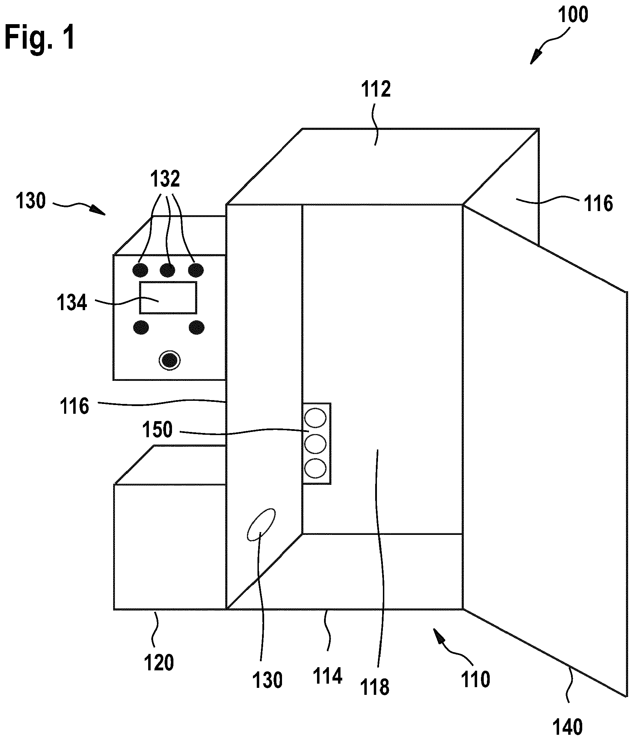

FIG. 1 shows a schematic perspective view of an apparatus 100 for sanitizing of an electromedical device (not shown) according to embodiments described herein.

The apparatus 100 includes a housing 110 having a reception space 118 configured for receiving the electromedical device, at least one nebulizer device 120 configured for nebulizing a sanitizing product (also referred to as "biocidal product"), and a controller 130 configured for controlling the at least one nebulizer device 120 for introducing a plurality of doses of the nebulized sanitizing product into the reception space 118 during a sanitization process. The reception space 118 is closeable. In particular, the reception space 118 can be closed during the sanitization process. The housing 110 can be a box or cabinet. In some implementations, the housing 110 can be made of metal, such as stainless steel or aluminum. As an example, the housing 110 can have an internal and external stainless steel structure.

According to the invention, which can be combined with the embodiments described herein, the sanitizing product is a hydrogen peroxide (H₂O₂) based sanitizing product having a hydrogen peroxide concentration between 4% and 10%, specifically between 6% and 8%, specifically between 6% and 7.9%, and more specifically of about 7.9%. The sanitizing product can be highly stabilized. The hydrogen peroxide concentration between 4% and 10% provides an effective sanitizing of the electromedical device while avoiding a damaging of the electromedical device, such as electronics and displays thereof. Further, a lifespan of the electromedical device is not reduced, since the sanitizing product having the hydrogen peroxide concentration does not affect materials and electronics of the electromedical device, contrary to e.g. ozone. In contrast thereto, a lifespan of the electromedical device can be significantly reduced when using an ozone based sanitizing product.

The reception space 118 has a volume, which can be enclosed by at least a part of the housing 110, such as walls of the housing 110. As an example, the housing 110 can include a top wall 112, a bottom wall 114, sidewalls 116, and a rear wall. The housing 110 can have at least one opening in the reception space 118 and at least one door 140, such as a front door, configured for closing the opening. The walls and optionally the closed door 114 can define the reception space 118, and can particularly define the volume of the reception space 118.

In some implementations, the reception space 118 can be substantially airtight. As an example, a sealing can be provided at the at least one opening such that the at least one door 140 can seal the opening substantially airtight. The sealing can be, for example, a rubber sealing such as a rubber band. The substantially airtight reception space 118 can reduce or even avoid a leakage of the nebulized sanitizing agent from the reception space 118 to the outside.

According to some embodiments, which can be combined with other embodiments described herein, the volume of the reception space 118 is about 5m³ or less, specifically about 3m³ or less, and more specifically about 1m³ or less. As an example, the volume can be in a range between about 1m³ and about 100m³, specifically in a range between about 1m³ and about 5m³, specifically in a range between about 2m³ and about 5m³, and can specifically be in the range between about 2m³ and about 3m³. The volume of the reception space can be 2.7m³.

In some implementations, each dose of the plurality of doses has, or corresponds to, between 0.5ml and 25ml of the sanitizing product per m³ of the volume of the reception space 118, and preferably between 15ml and 20ml of the sanitizing product per m³ of the volume of the reception space 118. The doses provide a sufficient amount of the sanitizing product per reception space volume to provide an effective sanitizing process. The nebulized sanitizing product can be distributed substantially uniformly within the reception space 118 such that the electromedical device is immersed in the mist of the sanitizing product for disinfection.

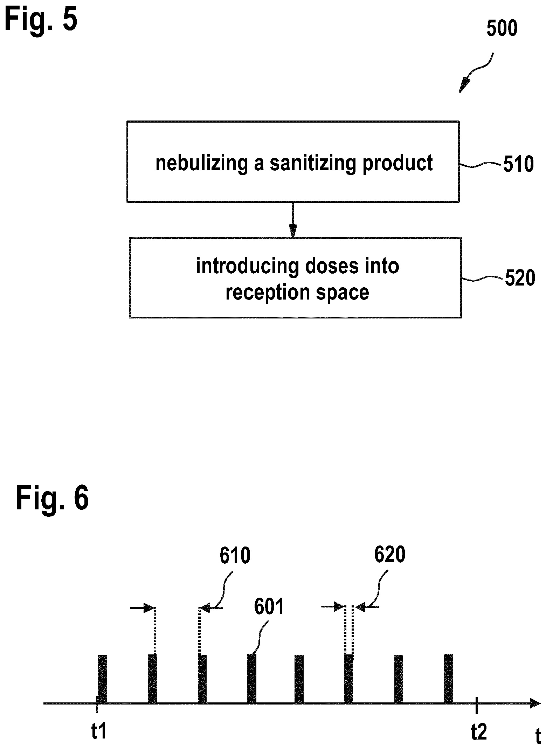

According to the invention, the controller 130 is configured to introduce the plurality of doses at predetermined time intervals into the reception space 118.The predetermined intervals can be between 1 seconds and 210 seconds, specifically between 90 seconds and 210 seconds, and particularly between 120 seconds and 180 seconds. In some embodiments, the predetermined intervals can be between 1 seconds and 5 seconds, and specifically between 3 and 4 seconds. in non-inventive embodiments, a controller can be configured to introduce the plurality of doses into a reception space continuously. In other words, the plurality of doses can be introduced without interruption therebetween. The controller 130 can further be configured to perform the sanitization process for a duration between 30 minutes and 90 minutes, and particularly for a duration of about 60 minutes. For example, see FIG. 6.

The controller 130 can include one or more input devices 132 and a display 134. As an example, the controller 130, and particularly the one or more input devices 132 and the display 134, can be include in an electric panel for automating the sanitization process (e.g. dosage and a contact time). Specifically, the one or more input devices 132 can allow a control of the apparatus 100, and particularly of the at least one nebulizer device. As an example, one or more process parameters, such as at least one of a duration of the sanitization process, a predetermined time interval between introduction of individual doses, a predetermined dosage amount, a temperature, a relative humidity, a pressure, and the hydrogen peroxide concentration in the sanitizing product can be inputted using the one or more input devices 132. The one or more process parameter are explained with respect to FIGs. 5 and 6 in greater detail. The display 134 can be configured to display information about the sanitization process, such as an operation state of the apparatus 100, a progress of the sanitization process, the one or more process parameters of the sanitization process, and the like.

The drawings illustrate the controller 130 as one single entity. However, the present disclosure is not limited thereto and portions of the controller 130 can be integrated elsewhere in the apparatus 100. As an example, a portion of the controller 130 can be included in the at least one nebulizer device 120.

According to some embodiments, the apparatus 100 includes optical signal devices, such as external lights (for example, red and green) to signal the current sanitization process and/or that the sanitization process is finished. Further, the apparatus 100 can include an emergency shutdown button, which can be provided by an input device of the one or more input devices 132. Moreover, an automatic switching can be provided, for example, when the at least one door 140 is opened and/or closed. As an example, the sanitization process can be stopped automatically when the at least one door 140 is opened e.g. during the sanitization process. Likewise, the sanitization process can be started automatically when the at least one door 140 is closed.

In some implementations, the at least one nebulizer device 120 is located inside or outside the housing 110 for introducing the nebulized sanitizing product into the reception space 118. According to the invention, the at least one nebulizer device 120 is located inside the reception space 118, any other configurations of the nebulizer device do not fall under the scope of the appended claims. As an example, the at least one nebulizer device 120 can be located outside both the housing 110 and the reception space 118, as it is illustrated in the example of FIG. 1. In other examples, the at least one nebulizer device 120 can be located inside the housing 110 but outside the reception space 118, e.g., in another reception space for the at least one nebulizer device 120. In such a case, the at least one nebulizer device 120 can be accessible from the outside of the housing. As an example, the housing 110 can have an opening in the other reception space through which the at least one nebulizer device 120 can be accessed. The housing 110 can have a door configured for closing the opening. It is to be understood that the location of the at least one nebulizer device 120 is not limited to these examples, and that the at least one nebulizer device 120 can be provided at other locations suitable for introduction of the nebulized sanitizing product into the reception space 118.

Although one nebulizer device is illustrated in the drawings, it is to be understood that the present disclosure is not limited thereto and that more than one nebulizer device can be provided. As an example, two or more nebulizer devices can be provided e.g. inside the reception space 118. The two or more nebulizer devices can be positioned such that the nebulized sanitizing product can be uniformly distributed within the reception space 118.

As used throughout the present disclosure, the at least one nebulizer device shall embrace devices capable of nebulizing the sanitizing product, which may be in a liquid form. In particular, the at least one nebulizer device 120 can disperse the liquid sanitizing product to generate mist, such as dry-mist, which is introduced into the reception space 118. The at least one nebulizer device 120 can be configured to adjust the dosage (ml/mc) to be nebulized. Further, the at least one nebulizer device 120 can be configured to micronize particles up to 30 µm. In some embodiments, the at least one nebulizer device 120 can be configured to record the sanitization process or cycle, for example, by printing or saving data on a memory.

According to some embodiments, which can be combined with other embodiments described herein, the reception space 118 includes one or more ports or openings 130 configured for introduction of the nebulized sanitizing product into the reception space 118. The one or more ports or openings 130 can be connected to the at least one nebulizer device 120, for example, via respective passages or lines. The passages or lines can be short to allow a proper introduction of the nebulized sanitizing product in the reception space 118. In some embodiments, the one or more ports or openings 130 can be provided in at least one wall of the housing 110, such as at least one of the sidewalls 116. Although FIG. 1 exemplarily illustrates one port or opening, more than one port or opening can be provided e.g. at different locations of the reception space 118 such that the nebulized sanitizing product can be uniformly introduced and distributed in the reception space 118.

The apparatus 100 further includes an electrical connection port 150 in the reception space 118. The electrical connection port 150 is configured for connection with the electromedical device to operate the electromedical device during the sanitization process. As an example, the electrical connection port 150 can include one or more electrical sockets or receptacles. In particular, the electrical connection port 150 can provide a power supply for the electromedical device. The number of electrical sockets can correspond to a number of electromedical devices that can be sanitized simultaneously. By operating the electromedical device during the sanitization process, the nebulized sanitizing product can be introduced in, and optionally circulated through, the electromedical device. As an example, if the electromedical device is a medical ventilator or respirator, internal components such as valves, tubes and pneumatic components can be sanitized. In other words, not only outer surfaces of the electromedical device are disinfected, but also internal components thereof. An effectiveness of the sanitization process can be improved.

According to some embodiments, which can be combined with other embodiments described herein, the apparatus 100 further includes at least one of an air extractor, an interior light, and one or more UVC lamps. As an example, the air extractor can remove the nebulized sanitizing product from the reception space 118 at the end of the sanitization process. The interior light can be provided in the reception space 118 for illumination of the reception space 118. The one or more UVC lamps can be used during at least a part of the sanitization process to further improve the sanitizing effectiveness.

The electromedical device according to the present disclosure shall embrace devices or equipment used for medical purposes, and which have electric means, such as electronics and/or displays. As an example, the electromedical device can be a medical ventilator or respirator for moving breathable air into and out of the lungs, to provide breathing for a patient who is physically unable to breathe, or breathing insufficiently. However, the present disclosure is not limited thereto and other electromedical devices, such as reusable electromedical devices, can be sanitized using the embodiments of the present disclosure.

The electromedical device is selected from the group consisting of a medical ventilator or respirator, high performances volume assured-pressure support pulmonary ventilators, pressure support ventilators, BiLevel devices, N-PAP devices, secretion clearance devices, IPPB (intermittent positive pressure breathing) devices, tracheal suction pumps, monitoring devices, antidecubitus compressors, heated humidifiers, and any combination thereof.

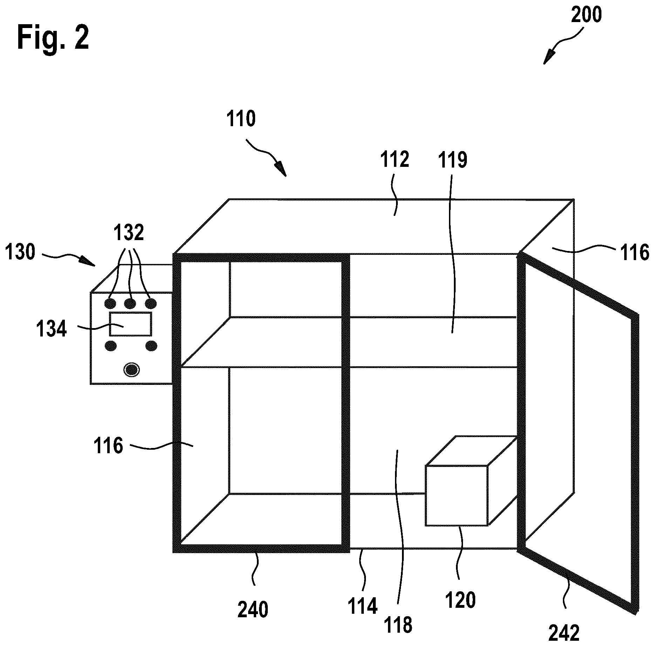

FIG. 2 shows a schematic perspective view of an apparatus 200 for sanitizing of an electromedical device according to further embodiments described herein. In the example of FIG. 2, the at least one nebulizer device 120 is located inside the reception space 118.

According to some embodiments, which can be combined with other embodiments described herein, the apparatus 200 includes one or more shelves 119 in the reception space 118. The electromedical device(s) can be positioned on the one or more shelves 118 during the sanitizing process. The apparatus 200 can be configured for simultaneously sanitizing of a plurality of electromedical devices. As an example, the plurality of electromedical devices can be positioned on the one or more shelves 119.

In some implementations, the apparatus 200 includes one or more doors, such as the single door 140 illustrated in FIG. 1 or the double doors illustrated in FIG. 2 having a first door 240 and a second door 242. In some implementations at least one door of the one or more doors can have a frame and a transparent inlay. As an example, the frame can be a metal frame and the transparent inlay can be glass. An operator of the apparatus 200 can watch the electromedical device(s) within the reception space 118, for example, during the sanitization process. In further implementations, the at least one door of the one or more doors can be a glass door.

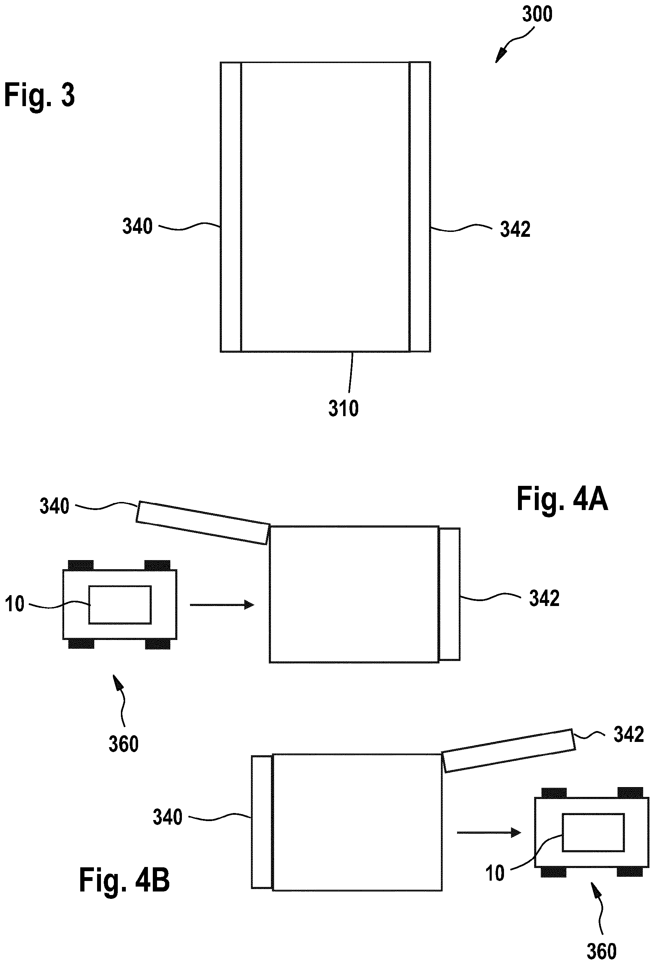

FIG. 3 shows a schematic top view of an apparatus 300 for sanitizing of an electromedical device 10 according to yet further embodiments described herein. FIGs. 4A and B show schematic top views of the apparatus of FIG. 3. The apparatus 300 has an in-out through tunnel structure or configuration.

According to some embodiments, the housing 310 of the apparatus 300 includes a first opening in the reception space and a second opening in the reception space, and a first door 340 configured for closing the first opening and a second door 342 configured for closing the second opening. The first opening and the second opening can be provided on opposite or adjacent sides of the reception space and/or the housing 310. Likewise, the first door 340 and the second door 342 can be provided on the opposite or adjacent sides of the reception space and/or housing 310.

The first opening (or entrance) can be configured for entry of the electromedical device 10 to be sanitized. The second opening (or exit) can be configured for an exit of the electromedical device 10 having been sanitized. As an example, the first door 340 can be an entry door and the second door 342 can be an exit door. In some implementations, the electromedical device 10 to be sanitized can be placed on a cart 360, for example, on shelves of the cart 360. The cart 360 can have wheels such that the cart 360 can be wheeled into the reception space through the first opening, as it is illustrated in FIG. 4A. The first door 340 can be closed and the sanitization process can be performed. After completion of the sanitization process, the second door 342 can be opened and the cart 360 having the sanitized electromedical device 10 positioned thereon can be wheeled out of the reception space through the second opening, as it is illustrated in FIG. 4B. The apparatus 300 of the present embodiment can allow for a quasi-continuous process flow for sanitizing contaminated electromedical devices.

FIG. 5 shows a flow chart of a method 500 for sanitizing an electromedical device according to embodiments described herein. FIG. 6 shows a sanitization process with multiple doses (or dosages) according to embodiments described herein.

According to embodiments described herein, the method 500 for sanitization of an electromedical device can be conducted by means of computer programs, software, computer software products and the interrelated controllers, which can have a CPU, a memory, a user interface, and input and output means being in communication with the corresponding components of the apparatus for sanitizing the electromedical device.

The method 500 includes in block 510 a nebulizing of a sanitizing product, and in block 520 an introducing of a plurality of doses (indicated with reference numeral 601 in FIG. 6) of the nebulized sanitizing product into a reception space having the electromedical device positioned therein.

According to some embodiments, which can be combined with other embodiments described herein, the electromedical device is operated during at least a part of the sanitization process, and particularly during essentially the entire duration of the sanitization process. As an example, the electromedical device can be operated during at least 50%, specifically during at least 75%, and more specifically during at least 90% of the duration of the sanitization process.

The sanitization process can be performed at least twice for a respective electromedical device. A first sanitization process can be performed for inbound electromedical devices e.g. coming from the territory ("inbound cycle"), and a second sanitization process can be performed after a technical maintenance process of the electromedical devices ("outbound cycle"). Performing the sanitization process at least twice can be particularly beneficial for electromedical devices that do not work (e.g. flow generators) or are not operable. In some implementations, operable electromedical devices, such as medical ventilators, can undergo the sanitization process only once.

According to some embodiments, which can be combined with other embodiments described herein, the sanitization process includes one or more process parameters selected from the group consisting of a duration of the sanitization process, a predetermined time interval between introduction of individual doses, a predetermined dosage amount, a temperature, a relative humidity, a pressure, the hydrogen peroxide concentration in the sanitizing product, and any combination thereof.

In some implementations, the duration of the sanitization process can be between about 30 minutes and about 90 minutes, specifically between about 45 and about 105 minutes, and can more specifically be about 60 minutes. The duration of the sanitization process can correspond to a duration for which the sanitizing of the electromedical device is performed. A shown in FIG. 6, the duration of the sanitization process can be defined between a start time t1 and an end time t2.

According to some embodiments, the predetermined time intervals 610 between introduction of individual doses can be between 90 seconds and 210 seconds, and particularly between 120 seconds and 180 seconds. The predetermined time intervals 610 can be defined between an end of the introduction of a dose and the beginning of the introduction of a subsequent dose. In particular, the introduction of a perspective dose can take a certain time. In other words, each introduction of a respective dose can be performed for a predetermined duration 620. The predetermined duration 620 of the dosage introduction can be selected such that a predetermined amount of the sanitization product (referred to as "predetermined dosage amount") is introduced into the reception space.

In some implementations, each dose of the plurality of doses has an amount between about 0.5±0.5ml and about 25±0.5ml of the sanitizing product per m³ of the volume of the reception space, and specifically between about 15±0.5ml and about 20±0.5ml of the sanitizing product per m³ of the volume of the reception space 118. The predetermined dosage amount can be defined as the amount of the sanitizing product per m³ times the volume of the reception space.

According to some embodiments, the sanitization process is performed at a temperature between 10°C and 40°C, and specifically at a temperature between 15°C and 30°C. The temperature can be the temperature within the reception space of the apparatus according to the present disclosure. In some implementations, the temperature can be essentially constant during the sanitization process. In other implementations, the temperature can be changed during the sanitization process. As an example, the temperature can be increased and/or decreased during the sanitization process.

In some embodiments, the sanitization process is performed at a relative humidity between 10% and 90%, and particularly at a relative humidity between 25% and 75%. The relative humidity is the ratio of the partial pressure of water vapor to the equilibrium vapor pressure of water at a given temperature. The relative humidity can be the relative humidity within the reception space of the apparatus at a given temperature. In some implementations, the relative humidity can be essentially constant during the sanitization process. In other implementations, the relative humidity can be changed during the sanitization process. As an example, the relative humidity can be increased and/or decreased during the sanitization process.

According to some embodiments, the sanitization process is performed at a pressure between 900mbar and 1100mbar, particularly at a pressure between 1005mbar and 1020mbar, and more particularly at atmospheric pressure. The pressure can be a pressure within the reception space of the apparatus according to the present disclosure. In some implementations, the pressure can be essentially constant during the sanitization process. In other implementations, the pressure can be changed during the sanitization process. As an example, the pressure can be increased and/or decreased during the sanitization process.

According to the invention, which can be combined with other embodiments described herein, the sanitizing product is a hydrogen peroxide based sanitizing product. The sanitizing product has a hydrogen peroxide concentration between about 4% and about 10%, specifically between about 6% and about 8%. As an example, the hydrogen peroxide concentration is about 7.9%. The hydrogen peroxide concentration between 4% and 10% provides an effective sanitizing of the electromedical device while avoiding a damaging of the electromedical device, such as electronics thereof. Further, a lifespan of the electromedical device is not reduced, since the sanitizing product having the hydrogen peroxide concentration does not affect materials and electronics of the electromedical device, contrary to e.g. ozone.

Process parameters for exemplary sanitization processes #1 to #4 are illustrated in the following table:

| # | interval [min] | dosage amount [ml] | dosage/m³ | duration [min] | nebulizer [ml/min] | volume reception space [m³] |
|---|---|---|---|---|---|---|
| 1 | 2 | 37 | 13.7 | 30 | 18.5 | 2.7 |
| 2 | 2 | 37 | 13.7 | 60 | 18.5 | 2.7 |
| 3 | 2.5 | 46.25 | 17.13 | 60 | 18.5 | 2.7 |
| 4 | 3 | 55.5 | 20.56 | 60 | 18.5 | 2.7 |

The above exemplary sanitization processes #1 to #4 can achieve an average log reduction of bacterial contamination, e.g., of Candida, Pseudomonas, Bacillus, and Staphylococci, by 3 to 5.

In the following, an exemplary sanitization process (also referred to as "sanitizing procedure") is described. First, the electromedical device can be registered before the sanitization process is initiated. The electromedical device can be pre-cleaned, for example, using paper towels with quaternary ammonium salt based products or benzalkonium chloride. The electromedical device can then be placed inside the reception space, for example, on its own shelf in the reception space (sanitation cabinet or container). Peroxide test strips can be attached e.g. at the four corners inside the reception space. As an example, for a reception space having a volume of less than 70m³, two or more additional strips can be attached inside the reception space, for example, at positions corresponding to half of the length of the reception space or area and e.g. 2m high.

If the electromedical device is configured for providing a flow or pressure, the electromedical device can be connected to power and switched on. For electromedical devices not included in this category, it can be sufficient to put said electromedical devices on the shelf. It can be tested whether the electromedical device works within normal parameters. As an example, a sanitizing efficiency would be reduced for electromedical devices that generate low flow or even no flow. If any of the electro medical devices are not working, e.g. a model and serial number thereof can be noted. Thereafter, the reception space can be closed and the sanitization process or cleaning cycle can be started. As an example, the sanitization process can set the one or more process parameters automatically. The sanitization process can include a dispensing of the micronized or nebulized sanitizing product. A dosage can be set to 17ml/mc, plus/minus 0.5. The spray can be homogenized into the air to allow dry aerosol to reach the inland areas and increase the time of contact with the surfaces, particularly of the electromedical device. Regardless of the operating time of the automated nebulizer, a total time of the sanitization process or cycle can be about 60 minutes. The operating time of the automated nebulizer can vary depending on the model used. However, an amount of the nebulized sanitizing product per m³ introduced into the reception space should be ensured.

At the end of the sanitization process, an air extractor can be activated for removing or evacuating the nebulized sanitizing product from the reception space to ensure safety when the doors are opened and to avoid danger to the operator. The opening of the reception space and the edges of the respective doors can be cleaned with paper towels with quaternary ammonium salt based products or benzalkonium chloride. This cleaning procedure can be performed for the apparatus described e.g. in FIGs. 1 and 2 (e.g. having no airtight sealing), and may not be performed for the apparatus is described in FIGs. 3, 4A and 4B (e.g. having an airtight sealing).

Now, the doors can be opened and the devices previously switched on can be shut down. The peroxide test strips can be checked to make sure that the hydrogen peroxide was correctly delivered during the sanitization process. As an example, if the test strips show negative or insufficient H₂O₂ dosage, the sanitization process should be repeated. The manual and/or operational instructions could be checked for troubleshooting.

Finally, the electromedical devices can be picked up and it can be recorded that the sanitizing has been performed, wherein the batch of the sanitizer used could be noted. Non-working devices could be left in the reception space and be subjected to another (second) sanitization process.

The present disclosure uses a reception space in which the electromedical device to be cleaned is positioned, wherein a plurality of doses of a nebulized sanitizing product including, for example, hydrogen peroxide, are introduced into the reception space during a sanitization process. The reception space is closed during the sanitization process. The electromedical device, and specifically internal parts thereof, such as ventilators, internal pneumatic circuits, tubes, and outlet flow connectors of medical ventilators, can be disinfected using the nebulized sanitizing product, such as a dry-mist hydrogen peroxide based sanitizing product. The embodiments of the present disclosure can achieve a reduction of bacterial contamination of more than 3 Log in just one sanitization process or cycle.

While the foregoing is directed to embodiments of the disclosure, other and further embodiments of the disclosure may be devised without departing from the basic scope thereof, and the scope thereof is determined by the claims that follow.

## Claims

1. An apparatus (100, 200, 300) for sanitizing of an electromedical device (10),
the electromedical device (10) being selected from a group consisting of a medical ventilator or respirator, high performances volume assured pressure support pulmonary ventilators, BiLevel devices, N-PAP devices, secretion clearance devices, tracheal suction pumps, monitoring devices, antidecubitus compressors, heated humidifiers, and any combination thereof
comprising:
a housing (110, 310) having a reception space (118) configured for receiving the electromedical device (10), wherein the reception space (118) has a volume;
at least one nebulizer device (120) configured for nebulizing a sanitizing product,
a controller (130) configured for controlling the at least one nebulizer device (120)
**characterized in that**
the apparatus further includes an electrical connection port (150) in the reception space (118), wherein the electrical connection port (150) is configured for being connected with the electromedical device (10) to operate the electromedical device (10) during the sanitization process,
the at least one nebulizer (120) is located inside the reception space (118) for introducing the nebulized sanitizing product into the reception space (118);
wherein the controller (130) is configured for introducing a plurality of doses (601) of the nebulized sanitizing product at predetermined time intervals (610) into the reception space (118) during a sanitization process, and
wherein the sanitizing product is a hydrogen peroxide based sanitizing product having a hydrogen peroxide concentration between 4% and 10%.

2. The apparatus (100, 200, 300) of claim 1, wherein the predetermined time intervals (610) are between 90 seconds and 210 seconds, and particularly between 120 seconds and 180 seconds.

3. The apparatus (100, 200, 300) of claim 1 or 2, wherein the controller (130) is configured to perform the sanitization process for a duration between 30 minutes and 90 minutes, and particularly for a duration of about 60 minutes.

4. The apparatus (100, 200, 300) of any one of claims 1 to 3, wherein the reception space (118) has a volume of 5m³ or less or 3m³ or less, particularly wherein the volume is between 2m³ and 3m³.

5. The apparatus (100, 200, 300) of any one of claims 1 to 4, wherein the housing (110, 310) has at least one opening in the reception space (118) and at least one door (140; 240, 242; 340, 342) configured for closing the opening.

6. The apparatus (300) of any one of claims 1 to 5, wherein the housing (310) further includes:
a first opening in the reception space and a second opening in the reception space; and
a first door (340) configured for closing the first opening and a second door (342) configured for closing the second opening,
wherein the first opening and the second opening are provided on opposite or adjacent sides of the reception space.

7. The apparatus (100, 200, 300) of any one of claims 1 to 6, wherein each dose of the plurality of doses (601) has between 0.5ml and 25ml of the sanitizing product per m³ of the volume of the reception space (118), and particularly between 15ml and 20ml of the sanitizing product per m³ of the volume of the reception space (118).

8. A method (500) for sanitizing of an electromedical device (10),
the electromedical device (10) being selected from a group consisting of a medical ventilator or respirator, high performances volume assured pressure support pulmonary ventilators, BiLevel devices, N-PAP devices, secretion clearance devices, tracheal suction pumps, monitoring devices, antidecubitus compressors, heated humidifiers, and any combination thereof, in a sanitizing apparatus (100, 200, 300) as claimed in any one of the preceding claims, comprising:
nebulizing (510) a sanitizing product being a hydrogen peroxide based sanitizing product and having a hydrogen peroxide concentration between 4% and 10%; and
introducing (520) a plurality of doses (601) of the nebulized hydrogen peroxide at predetermined time intervals (610) into a reception space having the electromedical device (10) therein, and
operating the electromedical device (10) during the sanitizing thereof.

9. The method (500) of claim 8, wherein the hydrogen peroxide concentration is between 6% and 8%, and particularly wherein the hydrogen peroxide concentration is 7.9%.

10. The method (500) of claim 8 or 9, wherein the predetermined time intervals are between 90 seconds and 210 seconds, and particularly between 120 seconds and 180 seconds.

11. The method (500) of any one of claims 8 to 10, wherein the sanitizing of the electromedical device (10) is performed at at least one or a combination of the following conditions:
for a duration between 30 minutes and 90 minutes, and particularly for a duration of about 60 minutes;
at a temperature between 10°C and 40°C, and particularly at a temperature between 15°C and 30°C;
at a relative humidity between 10% and 90%, and particularly at a relative humidity between 25% and 75%;
at a pressure between 900mbar and 1100mbar, particularly at a pressure between 1005mbar and 1020mbar, and more particularly at atmospheric pressure.

## Patentansprüche

1. Gerät (100, 200, 300) zum Desinfizieren einer elektromedizinischen Vorrichtung (10),
wobei die elektromedizinische Vorrichtung (10) aus einer Gruppe ausgewählt wird, die aus einem medizinischen Ventilator oder Respirator, volumengesicherten Hochleistungsbeatmungsgeräten mit Druckunterstützung, BiLevel-Vorrichtungen, N-PAP-Vorrichtungen, Sekretabsaugvorrichtungen, Trachealsaugpumpen, Überwachungsvorrichtungen, Antidekubitus-Kompressoren, beheizten Befeuchtungsgeräten und einer Kombination daraus besteht, wobei das Gerät Folgendes umfasst:
ein Gehäuse (110, 310), das einen Aufnahmeraum (118) aufweist, der konfiguriert ist, die elektromedizinische Vorrichtung (10) aufzunehmen, wobei der Aufnahmeraum (118) ein Volumen hat;
wenigstens eine Vernebelungsvorrichtung (120), die konfiguriert ist, ein Desinfektionsprodukt zu vernebeln,
eine Steuerung (130), die konfiguriert ist, die wenigstens eine Vernebelungsvorrichtung (120) zu steuern,
**dadurch gekennzeichnet, dass**
die Vorrichtung ferner einen Elektroanschluss (150) in dem Aufnahmeraum (118) umfasst, wobei der Elektroanschluss (150) so konfiguriert ist, dass er mit der elektromedizinischen Vorrichtung (10) verbunden wird, um die elektromedizinische Vorrichtung (10) während des Desinfektionsverfahrens zu betreiben,
wobei die wenigstens eine Vernebelungsvorrichtung (120) in dem Aufnahmeraum (118) angeordnet ist, um das vernebelte Desinfektionsprodukt in den Aufnahmeraum (118) einzubringen;
wobei die Steuerung (130) konfiguriert ist, während eines Desinfektionsverfahrens mehrere Dosen (601) des vernebelten Desinfektionsprodukts in festgelegten Zeitabständen (610) in den Aufnahmeraum (118) einzubringen, und
wobei das Desinfektionsprodukt ein Desinfektionsprodukt auf Wasserstoffperoxid-Basis ist, das eine Wasserstoffperoxid-Konzentration zwischen 4 % und 10 % hat.

2. Gerät (100, 200, 300) nach Anspruch 1, wobei die festgelegten Zeitabstände (610) zwischen 90 Sekunden und 210 Sekunden und vorzugsweise zwischen 120 Sekunden und 180 Sekunden liegen.

3. Gerät (100, 200, 300) nach Anspruch 1 oder 2, wobei die Steuerung (130) konfiguriert ist, das Desinfektionsverfahren für eine Dauer zwischen 30 Minuten und 90 Minuten und vorzugsweise für eine Dauer von etwa 60 Minuten durchzuführen.

4. Gerät (100, 200, 300) nach einem der Ansprüche 1 bis 3, wobei der Aufnahmeraum (118) ein Volumen von 5 m³ oder weniger oder von 3 m³ oder weniger hat, wobei das Volumen vorzugsweise zwischen 2 m³ und 3 m³ liegt.

5. Gerät (100, 200, 300) nach einem der Ansprüche 1 bis 4, wobei das Gehäuse (110, 310) wenigstens eine Öffnung in dem Aufnahmeraum (118) und wenigstens eine Tür (140; 240, 242; 340, 342), die zum Schließen der Öffnung konfiguriert ist, aufweist.

6. Gerät (300) nach einem der Ansprüche 1 bis 5, wobei das Gehäuse (310) ferner Folgendes umfasst:
eine erste Öffnung in dem Aufnahmeraum und eine zweite Öffnung in dem Aufnahmeraum; und
eine erste Tür (340), die konfiguriert ist, die erste Öffnung zu schließen, und eine zweite Tür (342), die konfiguriert ist, die zweite Öffnung zu schließen,
wobei die erste Öffnung und die zweite Öffnung an gegenüberliegenden oder angrenzenden Seiten des Aufnahmeraums vorgesehen sind.

7. Gerät (100, 200, 300) nach einem der Ansprüche 1 bis 6, wobei jede Dosis der mehreren Dosen (601) zwischen 0,5 ml und 25 ml Desinfektionsprodukt pro m³ Volumen des Aufnahmeraums (118) und vorzugsweise zwischen 15 ml und 20 ml Desinfektionsprodukt pro m³ Volumen des Aufnahmeraums (118) umfasst.

8. Verfahren (500) zum Desinfizieren einer elektromedizinischen Vorrichtung (10),
wobei die elektromedizinische Vorrichtung (10) aus einer Gruppe ausgewählt wird, die aus einem medizinischen Ventilator oder Respirator, volumengesicherten Hochleistungsbeatmungsgeräten mit Druckunterstützung, BiLevel-Vorrichtungen, N-PAP-Vorrichtungen, Sekretabsaugvorrichtungen, Trachealsaugpumpen, Überwachungsvorrichtungen, Antidekubitus-Kompressoren, beheizten Befeuchtungsgeräten und einer Kombination daraus besteht,
in einem Desinfektionsgerät (100, 200, 300) nach einem der vorhergehenden Ansprüche, wobei das Verfahren die folgenden Schritte umfasst:
Vernebeln (510) eines Desinfektionsprodukts, das ein Desinfektionsprodukt auf Wasserstoffperoxid-Basis ist und eine Wasserstoffperoxid-Konzentration zwischen 4 % und 10 % hat; und
Einbringen (520) von mehreren Dosen (601) des vernebelten Wasserstoffperoxids in festgelegten Zeitabständen (610) in einen Aufnahmeraum, in dem sich die elektromedizinische Vorrichtung (10) befindet, und
Betreiben der elektromedizinischen Vorrichtung (10) während der Desinfektion.

9. Verfahren (500) nach Anspruch 8, wobei die Wasserstoffperoxid-Konzentration zwischen 6 % und 8 % liegt und wobei die Wasserstoffperoxid-Konzentration vorzugsweise 7,9 % beträgt.

10. Verfahren (500) nach Anspruch 8 oder 9, wobei die festgelegten Zeitabstände zwischen 90 Sekunden und 210 Sekunden und vorzugsweise zwischen 120 Sekunden und 180 Sekunden liegen.

11. Verfahren (500) nach einem der Ansprüche 8 bis 10, wobei das Desinfizieren der elektromedizinischen Vorrichtung (10) unter wenigstens einer der folgenden Bedingungen oder einer Kombination daraus durchgeführt wird:
für eine Dauer zwischen 30 Minuten und 90 Minuten und vorzugsweise für eine Dauer von etwa 60 Minuten;
bei einer Temperatur zwischen 10 °C und 40 °C und vorzugsweise bei einer Temperatur zwischen 15 °C und 30 °C;
bei einer relativen Feuchtigkeit zwischen 10 % und 90 % und vorzugsweise bei einer relativen Feuchtigkeit zwischen 25 % und 75 %;
bei einem Druck zwischen 900 mbar und 1100 mbar, vorzugsweise bei einem Druck zwischen 1005 mbar und 1020 mbar, und insbesondere bei Atmosphärendruck.

## Revendications

1. Appareil (100, 200, 300) pour la désinfection d'un dispositif électromédical (10),
le dispositif électromédical (10) étant choisi parmi un groupe constitué d'un ventilateur médical ou d'un respirateur, de ventilateurs d'assistance pulmonaire à pression et volume contrôlés à hautes performances, de dispositifs à deux niveaux, de dispositifs N-PAP, de dispositifs de clairance des sécrétions, de pompes d'aspiration trachéales, de dispositifs de surveillance, de compresseurs anti-escarres, d'humidificateurs chauffés et de toute combinaison de ceux-ci
comportant
un caisson (110, 310) ayant un espace de réception (118) configuré pour recevoir le dispositif électromédical (10), dans lequel l'espace de réception (118) a un certain volume ;
au moins un dispositif de nébulisation (120) configuré pour nébuliser un produit désinfectant,
une commande (130) configurée pour commander le au moins un dispositif de nébulisation (120)
**caractérisé en ce que**
l'appareil inclut en outre un port de connexion électrique (150) dans l'espace de réception (118), dans lequel le port de connexion électrique (150) est configuré pour être connecté au dispositif électromédical (10) pour faire fonctionner le dispositif électromédical (10) pendant le processus de désinfection,
le au moins un dispositif de nébulisation (120) est situé à l'intérieur de l'espace de réception (118) pour introduire le produit désinfectant nébulisé dans l'espace de réception (118) ;
dans lequel la commande (130) est configurée pour introduire une pluralité de doses (601) du produit désinfectant nébulisé à des intervalles de temps prédéterminés (610) dans l'espace de réception (118) pendant un processus de désinfection, et
dans lequel le produit désinfectant est un produit désinfectant à base de peroxyde d'hydrogène ayant une concentration de peroxyde d'hydrogène comprise entre 4 % et 10 %.

2. Appareil (100, 200, 300) selon la revendication 1, dans lequel les intervalles de temps prédéterminés (610) sont compris entre 90 secondes et 210 secondes, et en particulier entre 120 secondes et 180 secondes.

3. Appareil (100, 200, 300) selon la revendication 1 ou 2, dans lequel la commande (130) est configurée pour exécuter le processus de désinfection pendant une durée comprise entre 30 minutes et 90 minutes, et en particulier pendant une durée d'environ 60 minutes.

4. Appareil (100, 200, 300) selon l'une quelconque des revendications 1 à 3, dans lequel l'espace de réception (118) a un volume de 5 m³ ou moins ou de 3 m³ ou moins, en particulier dans lequel le volume est compris entre 2 m³ et 3 m³.

5. Appareil (100, 200, 300) selon l'une quelconque des revendications 1 à 4, dans lequel le caisson (110, 310) a au moins une ouverture dans l'espace de réception (118) et au moins une porte (140 ; 240, 242 ; 340, 342) configurée pour fermer l'ouverture.

6. Appareil (300) selon l'une quelconque des revendications 1 à 5, dans lequel le caisson (310) inclut en outre :
une première ouverture dans l'espace de réception et une seconde ouverture dans l'espace de réception ; et
une première porte (340) configurée pour fermer la première ouverture et une seconde porte (342) configurée pour fermer la seconde ouverture,
dans lequel la première ouverture et la seconde ouverture sont agencées sur des côtés opposés ou adjacents de l'espace de réception.

7. Appareil (100, 200, 300) selon l'une quelconque des revendications 1 à 6, dans lequel chaque dose de la pluralité de doses (601) a entre 0,5 ml et 25 ml du produit désinfectant par m³ du volume de l'espace de réception (118), et en particulier entre 15 ml et 20 ml du produit désinfectant par m³ du volume de l'espace de réception (118).

8. Procédé (500) pour désinfecter un dispositif électromédical (10),
le dispositif électromédical (10) étant choisi parmi un groupe constitué d'un ventilateur médical ou d'un respirateur, de ventilateurs d'assistance pulmonaire à pression et volume contrôlés à hautes performances, de dispositifs à deux niveaux, de dispositifs N-PAP, de dispositifs de clairance des sécrétions, de pompes d'aspiration trachéales, de dispositifs de surveillance, de compresseurs anti-escarres, d'humidificateurs chauffés et de toute combinaison de ceux-ci, dans un appareil de désinfection (100, 200, 300) selon l'une quelconque des revendications précédentes, comportant les étapes consistant à :
nébuliser (510) un produit désinfectant étant un produit désinfectant à base de peroxyde d'hydrogène et ayant une concentration de peroxyde d'hydrogène comprise entre 4 % et 10 % ; et
introduire (520) une pluralité de doses (601) du peroxyde d'hydrogène nébulisé à des intervalles de temps prédéterminés (610) dans un espace de réception ayant le dispositif électromédical (10) dans celui-ci, et
faire fonctionner le dispositif électromédical (10) pendant la désinfection de celui-ci.

9. Procédé (500) selon la revendication 8, dans lequel la concentration de peroxyde hydrogène est comprise entre 6 % et 8 %, et en particulier dans lequel la concentration de peroxyde hydrogène est de 7,9 %.

10. Procédé (500) selon la revendication 8 ou 9, dans lequel les intervalles de temps prédéterminés sont compris entre 90 secondes et 210 secondes, et en particulier entre 120 secondes et 180 secondes.

11. Procédé (500) selon l'une quelconque des revendications 8 à 10, dans lequel la désinfection du dispositif électromédical (10) est réalisée à au moins une condition ou une combinaison des conditions suivantes :
pendant une durée entre 30 minutes et 90 minutes, et en particulier pendant une durée d'environ 60 minutes ;
à une température entre 10 °C et 40 °C, et en particulier à une température entre 15 °C et 30 °C;
à une humidité relative entre 10 % et 90 %, et en particulier à une humidité relative entre 25 % et 75 % ;
à une pression entre 900 mbar et 1 100 mbar, en particulier à une pression entre 1 005 mbar et 1 020 mbar, et plus particulièrement à la pression atmosphérique.
